# EUROPEAN PATENT SPECIFICATION

(11) **EP 3 302 627 B1**
(45) Date of publication and mention of the grant of the patent: **03.04.2019**
(21) Application number: 16726834.1
(22) Date of filing: 24.05.2016
(51) Int. Cl.: A61M 5/19, A61M 5/315

(54) **DRUG DELIVERY DEVICE**
ARZNEIMITTELABGABEVORRICHTUNG
DISPOSITIF D'ADMINISTRATION DE MÉDICAMENTS

(30) Priority: 27.05.2015 EP 15169310
(43) Date of publication of application: 11.04.2018
(73) Proprietor: Sanofi-Aventis Deutschland GmbH, 65929 Frankfurt am Main (DE)
(72) Inventor: GILMORE, Stephen Francis, Bristol BS3 1PT (GB); WEST, James, Bristol BS3 1PT (GB); COOP, James, Bristol BS3 1PT (GB); HAYTON, Paul, Bristol BS3 1PT (GB)
(74) Representative: Keil & Schaafhausen Patent- und Rechtsanwälte PartGmbB
(86) International application number: PCT/EP2016/061652
(87) International publication number: WO 2016/188984

(56) References cited:
- WO-A1-2012/072533

## Description

The present invention is directed to a drug delivery device for injecting two or more medicaments from separate medicament cartridges with a primary drug delivery assembly and a secondary drug delivery assembly.

Certain disease states require treatment using one or more different medicaments. Some drug compounds need to be delivered in a specific relationship with each other in order to deliver the optimum therapeutic dose. Here, combination therapy may be desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus. Another example of a medicament combination is the administration of a pain reliever in combination with a medicament for treating osteoarthritis. For example, a non-adjustable fixed dose of an anesthetic could be combined with an adjustable dose of an inflammatory medicament against, e.g. rheumatoid arthritis.

Drug delivery devices of the aforementioned kind often have applications where regular injection by persons without formal medical training occurs. This is increasingly common among patients having diabetes or the like, e.g. osteoarthritis. Self-treatment enables such patients to conduct effective management of their disease.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is applicable for both types of devices, i.e. for disposable devices as well as for reusable devices.

In combination therapy, a primary medicament and a secondary medicament are delivered in a specific relationship to deliver the optimum therapeutic dose. The injection devices of the generic kind usually comprise a housing in which two or more drug delivery assemblies are retained. Such devices include a primary drug delivery assembly for dispensing the primary medicament such as the long-acting insulin and a secondary drug delivery assembly for dispensing the secondary medicament, such as GLP-1. Some kinds of drug delivery assemblies comprise a compartment such as a cartridge holder for accommodating a replaceable medicament container such as a cartridge which stores the medicament.

In some cases, depending on the patient or the stage of the therapy, an effective treatment requires variations in the quantities and/or proportions of the medicaments making up the combined therapy. For example, the patient may require a non-adjustable fixed dose of the secondary medicament in combination with an adjustable variable dose of the primary medicament. For example, WO 2012/072533 A1 discloses a drug delivery device including a variable dose setting mechanism that is operably coupled to a primary reservoir holding a first medicament. The drug delivery device also includes a fixed dose setting mechanism that is operably coupled to a secondary reservoir holding a second medicament. The fixed dose setting mechanism comprises a trigger biasing member or spring. In addition, the drug delivery device includes a mechanical coupling that operably couples the variable dose setting mechanism and the fixed dose setting mechanism. During dose setting, activation of a single dose setter sets a variable dose of the first medicament. Setting of a minimum dose of the first medicament causes a fixed dose of the second medicament to be automatically set, and, during dispense, the trigger spring at least assists with the dispense of the fixed dose of the second medicament.

The effectiveness of a combined delivery of medicaments may require one or more doses to be delivered sequentially with one of the two medicaments being injected into the human body prior to the delivery of the other medicament. Such treatment may be conducted with devices that include two separate dispensing mechanisms that are actuated independently from each other such that the dispensing mechanisms are activated successively. However, such devices are difficult for users to handle.

Accordingly, there still exists a strong need to provide users of such injection devices with an easy to use device that allows safer and more convenient setting of two doses of medicaments stored in separate compartments.

The above object is solved by a drug delivery device with the features as defined in claim 1.

Drug delivery devices of the generic kind comprise a housing retaining a primary drug delivery assembly for the delivery of a primary medicament, wherein the primary drug delivery assembly comprises a primary dose setting member such as a dose setting sleeve, configured to rotate in a helical movement in proximal direction, usually in a first rotational direction, to set a dose of the primary medicament to be dispensed. When a dose of the primary medicament has not been set, the primary dose setting member is in a zero unit dose position, the most distal position of the primary dose setting member. The drug delivery device further comprises a secondary drug delivery assembly for the delivery of a secondary medicament, wherein the secondary drug delivery assembly comprises a secondary dose setting member, such as a dosing sleeve, configured to move axially in a proximal direction to set a dose of the secondary medicament.

According to the invention, a dose dial, preferably configured as a dial collar, is provided that is movable between a first position and a second position in axial direction with respect to the primary dose setting member, wherein the dial dose is configured in such way that the dose dial is rotationally constrained with respect to the housing when in the first position and when moved from the first to the second position when the primary dose setting member is in a zero unit dose position and in such way that the dose dial is rotationally constrained to the primary dose setting member in the second position (respectively when the dose dial is in the first position and moved from the first into the second position) and free to rotate relative to the housing. A bridging component is axially constrained to the secondary dose setting member and is coupled to the dose dial in such way that the bridging component is axially constrained to the dose dial when the dose dial is moved from the first into the second position such that a dose of the secondary medicament is set, and in such way that rotation of the dose dial in the second position to set a dose of the primary medicament causes the dose dial to move relative to the bridging component in axial direction so that further dose setting of the secondary medicament is prevented.

By means of the dose dial, the user can use a pulling action to set a dose of the secondary medicament, wherein axial movement of the dose dial during that first phase does not cause any dose setting in the primary drug delivery assembly. The dose dial may be splined to the housing until the dose dial reaches the second position. When the dose of the secondary medicament is set, the dose dial is rotationally coupled to the primary dose setting member. By rotation of the dose dial, the user can set the dose of the primary medicament to be dispensed. The dose dial may also be permanently keyed to the primary dose setting member.

The dose dial may also be provided with spline features such as teeth that engage corresponding spline features on the primary dose setting member in second position. The secondary dose setting member is preferably configured to rotate along a helical pattern like the primary dose setting member in proximal direction for setting of an increasing dose.

The housing may retain the drug deliver assemblies, with the drug delivery assemblies being placed next to each other. At a distal section, medicament cartridges with the two medicaments may be attached. The drug delivery assemblies respectively comprise a piston rod, a lead screw or the like configure to displace in distal direction during dispense such as to displace a bung in the respective medicament cartridge. A "2 to 1" needle adapter may be attached to the housing. In a preferred embodiment, this adapter is a disposable component; however, it is expediently designed so that it can be reused for multiple injections before it must be discarded. The delivery needle may be replaced after every injection. The delivery needle may attach to a hub that is integral to the adapter. The adapter includes two needles that are respectively configured for fluid communication with one of the two medicament cartridges and the injection needle.

Suitable drug delivery assemblies that may be retained in the housing are described in EP 1 603 610 A1, which is incorporated herein by reference. In such device, modifications as describes in the following are possible.

The single drug delivery assembly comprises a piston rod with a first threaded portion at a first (distal) end and a second threaded portion at a second (proximal) end. The first thread and the second thread are oppositely disposed. An insert is provided with a threaded insert, which is in threaded engagement with the first threaded portion of the piston rod.

A drive sleeve extends about the piston rod. The drive sleeve is generally cylindrical. The drive sleeve is provided at a first (distal) end with a first radially extending flange. A second radially extending flange is provided spaced a distance along the drive sleeve from the first flange. An intermediate thread is provided on an outer part of the drive sleeve extending between the first flange and the second flange. A helical groove extends along the internal surface of the drive sleeve. The second thread of the piston rod is adapted to work within the helical groove.

A shoulder is formed between a proximal end of the drive sleeve and an extension provided at the proximal end of the drive sleeve. The extension has reduced inner and outer diameters in comparison to the remainder of the drive sleeve. A second end of the extension is provided with a radially outwardly directed flange.

A clutch is disposed about the drive sleeve, between the drive sleeve and a dose setting member, which is configured as a dose dial sleeve. The dose dial sleeve rotates in a helical movement, a combination of rotation and axial displacement during dose setting. The dose dial sleeve is provided outside of the clutch and radially inward of the main housing. A helical groove is provided about an outer surface of the dose dial sleeve engaging a helical rib provided by the housing. The helical groove on the dose dial sleeve and the helical groove in the drive sleeve have the same lead. This allows the dose dial sleeve to extend from the main housing and the drive sleeve to climb the piston rod at the same rate

The clutch is cylindrical and is provided at a distal end with a series of saw teeth. Towards the proximal end of the clutch means there is located a radially inwardly directed flange. The flange of the clutch means is disposed between the shoulder of the drive sleeve and the radially inwardly directed flange of the extension. The proximal end of the clutch means is provided with a plurality of dog teeth adapted to engage with a second end of the dose dial sleeve. The clutch is keyed to the drive sleeve by way of splines to prevent relative rotation between the clutch and the drive sleeve.

A clicker spring is located adjacent the proximal flange of the drive sleeve and splined to the housing. The clicker spring has a flexible arm extending in proximal direction, engaging the saw teeth of the clutch. A dose dial grip is secured to the dose dial sleeve to prevent relative movement therebetween. The dose dial grip is provided with a central opening. A dispense button of generally 'T' section is provided at a second end of the pen-type injector. A stem of the button may extend through the opening in the dose dial grip

Dose setting is performed by rotation of the dose dial which rotates the dose dial sleeve. When the desired dose has been dialed, the user may then dispense this dose by depressing the button. The button engages and displaces the clutch axially with respect to the dose dial sleeve causing the dog teeth to disengage. However the clutch means remains keyed in rotation to the drive sleeve. The dose dial sleeve and associated dose dial grip are now free to rotate (guided by the helical rib located in helical groove).

The axial movement deforms the flexible arm of the clicker spring and ensures that the saw teeth cannot be overhauled during dispense. This rotationally locks the clutch to the main housing and prevents the drive sleeve from rotating with respect to the main housing though it is still free to move axially with respect thereto. This deformation is subsequently used to urge the clutch back into proximal direction to restore the connection between the clutch and the dose dial sleeve when pressure is removed from the button.

The longitudinal axial movement of the drive sleeve causes the piston rod to rotate through the opening in the insert, thereby to advance the piston in the cartridge.

In the sense of the invention, the dial grip of the above described drug delivery assembly may form the respective dose setting member. Of course, the dose dial may also form the dose setting member.

According to a further embodiment of the invention, the dose dial is configured as a sleeve at least partly surrounds the primary dose setting member.

According to a further embodiment of the invention, the dose dial is axially splined to the housing in the first position and is splined to the primary dose setting member in the second position. This provides extra safety in terms of not dialing a dose of the primary medicament in the first phase of the dose setting. A flexing arm on the housing may lock the dose dial against rotation, wherein the dose dial disengages from the flexing arm in the second position.

In order to couple the bridging component to the primary dose setting member, the dose dial may have an outer thread extending helically in axial direction and the bridging component is in threaded engagement with the outer thread of the dose dial. Rotation of the dose dial relative to the bridging component does not cause displacement of the bridging component and the user is free to set an increasing dose of the primary medicament without influencing the set dose of the secondary medicament. The thread engagement between the dose dial and the bridging component may have the same lead as the thread engagement between the primary dose setting member and the housing body.

According to a further embodiment of the invention, the secondary drug delivery assembly comprises a spring configured to bias the secondary dose setting member in proximal direction. The spring may support the setting of the dose of the secondary medicament.

For determining a fixed dose that cannot be influenced by the user in the combined therapy treatment, a stop may be provided to limit the axial travel of the bridging component in proximal direction, wherein the bridging component reaches the stop when the dose dial reaches the second position.

According to a further embodiment, the drug delivery device comprises a primary medicament cartridge and a secondary medicament cartridge, wherein the primary medicament cartridge is coupled to the primary drug delivery assembly and the secondary medicament cartridge is coupled to the secondary drug delivery assembly, and wherein the primary and the secondary medicament cartridge contain a medicament. The cartridges may be accommodated in an at least partly transparent cartridge holder.

According to a further embodiment of the invention, the drug delivery device is configured as a disposable device.

According to a further embodiment of the invention, the secondary drug delivery assembly comprises a drive sleeve being releasably connected to the secondary dose setting member and threaded to a piston rod such that pure axial motion of the drive sleeve causes the piston rod to rotate, wherein the piston rod is in threaded engagement with the housing or a housing insert. Such assembly may be configured as described in EP 1 603 610 A1 and is basically the same as the primary drug delivery assembly. A clicker spring is provided to rotationally lock the drive sleeve for dose dispensing, wherein the clicker spring is configured to be rotationally locked to the housing when a predetermined minimum dose of the secondary medicament is set. For that purpose, the clicker spring may comprise splines, teeth or the like, configured to engage corresponding splines or grooves on the housing which rotationally lock the clicker spring. The housing may comprise a section where the clicker spring is free to rotate. The clicker spring engages the groove of the housing, when the secondary dose setting member reaches the stop. As a result, the clicker spring is locked against rotation. This mechanism ensures that a dose under a minimum dose cannot be dispensed from the secondary drug delivery assembly.

To ensure that the secondary medicament is dispensed prior to the primary medicament, according to a further embodiment of the invention, a dose button is arranged in the dose dial in such way that displacement of the dose button in distal direction causes the dose dial to move in distal direction from the second into the first position such that a set dose of the secondary medicament is dispensed and in such way that when the dose dial is in the first position, the dose button engages a dispense button of the primary drug delivery assembly such that further movement of the dose button causes the set dose of the primary medicament to be dispensed.

The primary dose setting member may be configured as a number sleeve with a dose scale provided thereon. For indicating the set doses, the number sleeve dose scale may incorporate two number rows with one number row indicating the set dose of the primary medicament and with the other number row indicating the set dose of the secondary medicament.

The device efficiently prevents mono-dosing of the primary and of the secondary medicament and ensure that the patient receives a fixed dose of the secondary medicament before the primary medicament can be administered.

The device efficiently facilitates that the medicaments are administered one after the other thus preventing mixing with one another. This ensures that each medicament provides its intended effect alone without interacting with each other.

The effect that the non-adjustable fixed dose is administered before the adjustable dose is particularly beneficial for example when a non-adjustable fixed dose of an anesthetic is administered to provide pain relief and is combined with an adjustable dose of an inflammatory medicament against, e.g. rheumatoid arthritis. Thus the painful administration of a medicament can be mitigated with administering a local anesthetic beforehand. Another example would be a combination therapy for patients suffering from diabetes that require a fixed dose of a GLP-1 and an individual dose of a long-acting insulin in a combination therapy.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 Exendin-4(1-39),
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
des Pro36 [Met(O)14 Trp(O2)25, IsoAsp28] Exendin-4(1-39); or
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
   des Pro36 Exendin-4(1-39)-Lys6-NH2 (AVE0010),
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(O2)25, Asp28] Exendin-4(1-39)-Lys6-NH2,
   H-des Asp28 Pro36, Pro37, Pro38 [Trp(O2)25] Exendin-4(1-39)-NH2,
   H-(Lys)6-des Pro36, Pro37, Pro38 [Trp(O2)25, Asp28] Exendin-4(1-39)-NH2,
   H-Asn-(Glu)5-des Pro36, Pro37, Pro38 [Trp(O2)25, Asp28] Exendin-4(1-39)-NH2,
   des Pro36, Pro37, Pro38 [Trp(O2)25, Asp28] Exendin-4(1-39)-(Lys)6-NH2,
   H-(Lys)6-des Pro36, Pro37, Pro38 [Trp(O2)25, Asp28] Exendin-4(1-39)-(Lys)6-NH2,
   H-Asn-(Glu)5-des Pro36, Pro37, Pro38 [Trp(O2)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,

   H-Asn-(Glu)5-des Pro36, Pro37, Pro38 [Met(O)14, Trp(O2)25, Asp28] Exendin-4(1-39)-NH2, des Pro36, Pro37, Pro38 [Met(O)14, Trp(O2)25, Asp28] Exendin-4(1-39)-(Lys)6-NH2, H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids. There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Non-limiting, an exemplary embodiment of the invention will now be described with reference to the accompanying drawings, in which
- Fig. 1: shows a the dual cartridge drug delivery device in accordance with a first embodiment of the invention in a perspective view with parts removed; and
- Fig. 2: a distal section of the secondary drug delivery assembly of the drug delivery device in figure 1 in accordance with a further embodiment of the invention.

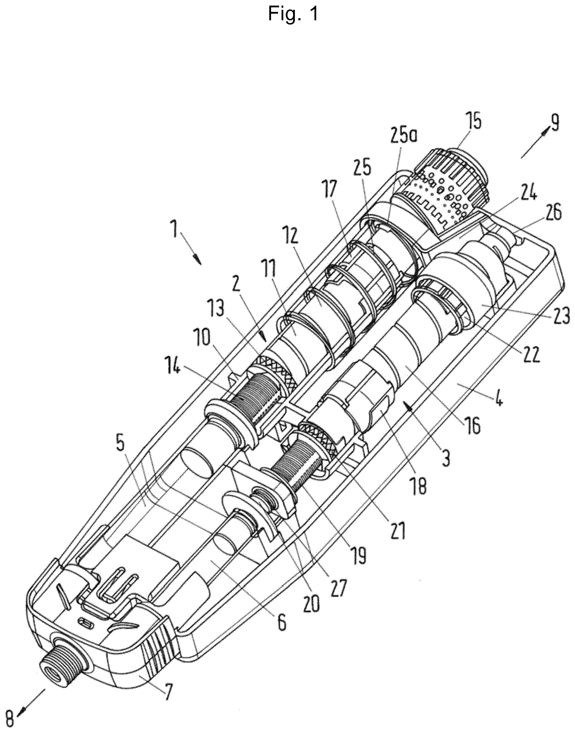

Figure 1 shows the interior of a drug delivery device 1 comprising a primary drug delivery assembly 2 and a secondary drug delivery assembly 3, both retained in a housing 4. The primary drug delivery assembly 2 serves for the delivery of a primary medicament contained in a primary cartridge 5. The secondary drug delivery assembly 3 serves for the delivery of a secondary medicament contained in a secondary cartridge 6. The cartridges may also be retained in a cartridge holder that is attachable to the distal end of the device 1. The cartridge holders for the cartridges may be integrated in a single cartridge holder hub.

At the distal end of the assembly, a 2-1 needle adapter 7 is attached. At the distal end 8 of the needle adaptor 7 a screw thread is formed. An injection needle can be attached to said screw thread or injection. The injection needle is in fluid communication with two proximally oriented needles in the needle adapter 7 that pierce the respective septa of the respective medicament cartridge 5 and 6. The device 1 extends from the distal end 8 to a proximal end 9.

The construction of the two drug delivery assemblies 2, 3 is basically the same. The primary drug delivery assembly 2 comprises a primary housing, housing body or housing section 10, a primary dose setting member 11 in the form of a dose dial sleeve, which is threadedly engaged with a threaded insert 17 fixed in the primary housing section 10 so that when the primary dose setting member 11 is rotated, it moves along a helical pattern in axial direction. The primary dose setting member 11 is connected to a dose dial 12, which is configured as a dial collar and which assembles as a sleeve over the primary drug delivery assembly 2.

The dose 12 is movable between a first distal axial position and a second proximal axial position relative to the primary dose setting member 11. The dose dial 12 is rotationally constrained with respect to the housing 4 when in the first position and when the dose setting member 11 is in its most distal position, which corresponds to a set dose of zero units. For that purpose, the housing 4 has on its inner surface a number of axially extending splines (not shown) or an axially extending flexing rib engaging a spline or a number of splines on the dose dial 12. When the dose dial 12 is moved proximally in axial direction, it disengages from the splined connection with the housing 4 and engages with a corresponding spline connection provided on the dial sleeve (primary dose setting member) 11 so that the dose dial 12 is rotationally constrained to the primary dose setting member 11. Rotation of the dose dial 12 is then transferred to the primary dose setting member 11. The primary dose setting member 11 is usually rotated (in a first rotational direction) to set an increasing dose of the primary medicament. When the dose dial 12 is in the second position, a dose of the primary medicament is set by rotating the dose dial 12. The dose dial 12 and the primary dose setting member 11 displace in proximal direction in a helical movement.

Inside the primary drug delivery assembly 2, there is a dose dispense mechanism including a clutch 13, a piston rod and a drive sleeve 14. For dispense a dose button 15 is pressed. The dose button 15 is urged in proximal direction 9 by a spring (not shown) away from a dispense button (not shown) of the primary drug delivery assembly 2.

When a dose of a primary medicament has been set, the dose button 15 is pressed so that the dose button acts on the dispense button of the primary drug delivery assembly. The dispense button acts on the clutch mechanism between the primary drive sleeve 14 and the primary dose setting member 11 which is disengaged and the primary dose setting member 11 is forced to move in distal direction in a pure axial motion thereby rotating the piston rod located inside. As the piston rod is in threaded engagement with a housing insert, the piston rod displaces in distal direction thereby displacing a bung in the cartridge so that the set dose of the primary medicament is dispensed.

The inner structure of the secondary drug delivery assembly 3 is very similar to that of the primary drug delivery assembly 2. The secondary drug delivery assembly 3 also comprises a secondary dose setting member 16 that is in threaded engagement with a threaded insert 18 fixed in a secondary housing, housing section or housing body.

Located distally (in dispensing direction) from the secondary dose setting member 16, a secondary drive sleeve 19 is located, which is in threaded engagement with the secondary piston rod 20. A clutch 21 is disposed between the secondary drive sleeve 19 and the secondary dose setting member 16. At the proximal end of the secondary drug delivery assembly 3, a secondary dose dial grip 22 is rotationally constrained to the secondary dose setting member 16. The secondary dose dial grip 22 is rotationally and axially constrained to cap member 23, that is axially constrained to a bridging component 24 but which can rotate relative thereto. The bridging component 24 is axially guided inside the housing 4 by means of a splined groove connection (not shown). The bridging component 24 surrounds the dose dial 12 and has on an inner surface an inner thread which engages an outer thread 25 on the dose dial 12 so that the bridging component 24 and the dose dial 12 are in threaded engagement 25a. Rotation of the dose dial 12 relative to the bridging component 24 causes relative axial displacement thereof.

In order to set a dose, the user is forced to follow specific steps. As the dose dial 12 is blocked rotationally within the housing 4 until it is pulled upwards (proximally) from the first position into the second position relative to the primary dose setting member 11, the user has to pull the dose dial 12 in proximal direction. That causes the splined engagement with the housing 4 to be removed. Thereafter, the dose dial 12 can be rotated wherein this rotation is transmitted to the primary dose setting member 11 such that a dose of the primary medicament is set. When the dose dial 12 is pulled in proximal direction, the bridging component 24 moves with the dose dial 12 by virtue of its threaded engagement in proximal direction.

This pull-to-activate user step forces the secondary dose setting member 16 to move in proximal direction as well. The secondary dose setting member 16 can rotate relative to the bridging component 24. As the secondary dose setting member 16 travels proximally, a dose of the secondary medicament is set. As the dose of the secondary medicament is set, a dose of the primary medicament is not set because the dose dial 12 moves from its first position to its second position without rotating or moving the primary dose setting member 11 in proximal direction. When the dose dial 12 reaches its second position, the dose dial 12 is free to rotate. A stop 26 abuts the bridging component 24 so that the bridging component 24 cannot be displaced further in proximal direction. In the second position, which corresponds to the abutment of the bridging component 24 at the stop 26, the dose dial 12 is free to rotate. The user is now allowed to rotate the dose dial 12 to set an increasing dose of the primary medicament. Rotation of the dose dial 12 does not cause displacement of the bridging component 24 in proximal direction so that there is no further dose setting in the secondary drug delivery assembly 3.

The device in figure 1 has a modification in the secondary drug delivery assembly 3 in comparison to the primary drug delivery assembly 2. A compression spring 27 urges the secondary drive sleeve 19 in proximal direction and automatically pushes the secondary drive sleeve 19 and accordingly the secondary dose setting member 16 in proximal direction when the dial grip 12 is pulled proximally. This feature allows implying a detent action (not shown) to retain the dose dial 12 until the user applies some axial force. Thereafter, the spring 27 sends the mechanism to the set stop 26 so that the device is ready for dialing the variable dose of the primary medicament.

The depression of the dose button 15 displaces the dose dial 12 and in turn the bridging component 24. The spring that urges the dose button in proximal direction applies a force in that is stronger than a force that is required to displace the bridging component 24. Movement of the bridging component 24 in distal direction acts on a dispense button in the secondary drug delivery assembly. This declutches the clutch mechanism in the secondary drug delivery assembly 3 so that dispense of the secondary medicament is initiated.

The dose button 15 reaches the primary dispense button, when the bridging component 24 is moved to its original initiate position. Then the dose button 15 engages the primary dispense button and initiates dispense of the primary medicament by declutching the clutch inside the primary drug delivery assembly 2. Further displacement of the dose dial 12 by depressing the dose button 15 causes the primary dose setting member 11 to rotate back in distal direction thereby dispensing the primary medicament. Accordingly, the fixed dose of the secondary medicament is dispensed prior to the variable dose of the primary medicament. The dose dial rotates through the thread engagement 25a with the bridging component and moves relative to the bridging component in distal direction.

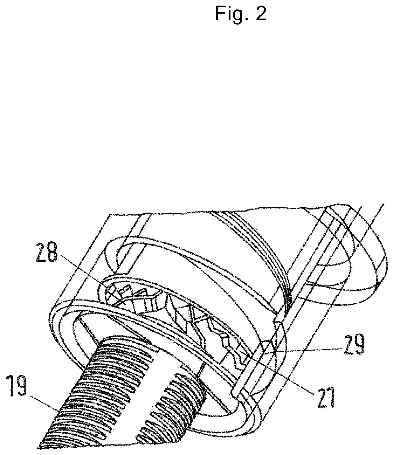

Figure 2 show a part of the secondary drug delivery assembly with the inner clutch 21, the secondary drive sleeve 19, a clicker spring 28 and a vertical rib 29 provided on the secondary housing. During the setting of a dose in the secondary drug delivery assembly, the clicker spring 28 rotates with the clutch 21. The spring is in clearance during a full 180°- rotation and comes to rest aligned with the vertical rib 29. When the user now commits to dispense a dose, the clutch 21 is displaced compressing the spring 28. This causes the spring 28 to become rotationally locked-out against the vertical rib 29. For the axial travel of the clutch 21, the spring 28 and the secondary drive sleeve 19 and advance the piston rod in the as described above.

### Reference numerals

- 1: drug delivery device
- 2: primary drug delivery assembly
- 3: secondary drug delivery assembly
- 4: housing
- 5: primary cartridge
- 6: secondary cartridge
- 7: needle adapter
- 8: distal end
- 9: proximal end
- 10: primary housing
- 11: primary dose setting member (primary dose dial sleeve)
- 12: dose dial
- 13: primary clutch
- 14: primary drive sleeve
- 15: dose button
- 16: secondary dose setting member
- 17: threaded insert of the primary drug delivery assembly
- 18: threaded insert of the secondary drug delivery assembly
- 19: secondary drive sleeve
- 20: secondary piston rod
- 21: secondary clutch
- 22: secondary dose dial grip
- 23: cap member
- 24: bridging component
- 25: outer thread on dose dial
- 25a: threaded engagement
- 26: stop
- 27: compression spring
- 28: clicker spring
- 29: vertical rib

## Claims

1. A drug delivery device (1) comprising:
a housing (4) retaining a primary drug delivery assembly (2) for the delivery of a primary medicament, wherein the primary drug delivery assembly (2) comprises a primary dose setting member (11) configured to rotate in a helical movement in proximal direction (9) to set a dose of the primary medicament;
and a secondary drug delivery assembly (3) for the delivery of a secondary medicament, wherein the secondary drug delivery assembly (3) comprises a secondary dose setting member (16) configured to move in proximal direction (9) to set a dose of the secondary medicament,
**characterized by** a dose dial (12) movable between a first position and a second position in axial direction with respect to the primary dose setting member (11), wherein the dose dial (12) is configured in such way that the dose dial (12) is rotationally constrained with respect to the housing (4) when moved from the first position into the second position when the primary dose setting member (11) is in a zero unit dose position and in such way that the dose dial (12) is rotationally constrained to the primary dose setting member (11) in the second position and is free to rotate relative to the housing (4),
wherein a bridging component (24) is axially constrained to the secondary dose setting member (16) and is coupled to the dose dial (12) in such way that the bridging component (24) is axially constrained to the dose dial (12) when the dose dial (12) is moved from the first into the second position such that a dose of the secondary medicament is set, and in such way that rotation of the dose dial (12) in the second position to set a dose of the primary medicament causes the dose dial (12) to move relative to the bridging component (24) in axial direction.

2. Drug delivery device according to claim 1, wherein the dose dial (12) is configured as a sleeve at least partly surrounds the primary dose setting member (11).

3. Drug delivery device according to claim 1 or 2, wherein the dose dial (12) is axially splined to the housing (4) in the first position and is splined to the primary dose setting member (11) in the second position.

4. Drug delivery device according to any of the preceding claims, wherein the dose dial (12) has an outer thread (25) extending helically in axial direction and wherein the bridging component (24) is in threaded engagement (25a) with the outer thread (25) of the dose dial (12).

5. Drug delivery device according to any of the preceding claims,
wherein the secondary drug delivery assembly (3) comprises a spring (27) configured to bias the secondary dose setting member (16) in proximal direction.

6. Drug delivery device according to any of the preceding claims, wherein a stop (26) is provided to limit the axial travel of the bridging component (24) in proximal direction, wherein the bridging component (24) reaches the stop (26) when the dose dial (12) reaches the second position.

7. Drug delivery device according to any of the preceding claims comprising a primary medicament cartridge (5) and a secondary medicament cartridge (6), wherein the primary medicament cartridge (5) is coupled to the primary drug delivery assembly (2) and the secondary medicament cartridge (6) is coupled to the secondary drug delivery assembly (3), and wherein the primary and the secondary medicament cartridge (5, 6) respectively contain a medicament.

8. Drug delivery device according to any of the preceding claims, wherein the drug delivery device (1) is configured as a disposable device.

9. Drug delivery device according to any of the preceding claims, wherein the secondary drug delivery assembly (3) comprises a drive sleeve (19) being releasably connected to the secondary dose setting member (16) and threaded to a piston rod (20) such that pure axial motion of the drive sleeve (19) causes the piston rod (20) to rotate, wherein the piston rod (20) is in threaded engagement with the housing or a housing insert,
wherein a clicker spring (28) is provided to rotationally lock the drive sleeve (19) for dose dispensing, wherein the clicker spring (28) is configured to be rotationally locked to the housing when a predetermined minimum dose of the secondary medicament is set.

10. Drug delivery device according to any of the preceding claims, comprising a dose button (15) arranged in the dose dial (12) in such way that displacement of the dose button (15) in distal direction causes the dose dial (12) to move in distal direction from the second into the first position and such that when the dose dial (12) is in the first position, the dose button (15) engages a dispense button of the primary drug delivery assembly (2) such that further movement of the dose button (15) causes a set dose of the primary medicament to be dispensed.

## Patentansprüche

1. Medikamenten-Verabreichungsvorrichtung (1), umfassend:
ein Gehäuse (4), das eine primäre Medikamenten-Verabreichungsanordnung (2) zur Verabreichung eines primären Medikaments zurückhält, wobei die primäre Medikamenten-Verabreichungsanordnung (2) ein primäres Dosiseinstellelement (11) umfasst, das dazu ausgelegt ist, sich in einer spiralförmigen Bewegung in proximaler Richtung (9) zu drehen, um eine Dosis des primären Medikaments einzustellen;
und eine sekundäre Medikamenten-Verabreichungsanordnung (3) zur Verabreichung eines sekundären Medikaments, wobei die sekundäre Medikamenten-Verabreichungsanordnung (3) ein sekundäres Dosiseinstellelement (16) umfasst, das dazu ausgelegt ist, sich in proximaler Richtung (9) zu bewegen, um eine Dosis des sekundären Medikaments einzustellen, **gekennzeichnet durch** eine Dosiswahlvorrichtung (12), die zwischen einer ersten Position und einer zweiten Position in axialer Richtung bezüglich des primären Dosiseinstellelements (11) bewegbar ist, wobei die Dosiswahlvorrichtung (12) derart ausgelegt ist, dass die Dosiswahlvorrichtung (12) bezüglich des Gehäuses (4) drehfest gehalten wird, wenn sie aus der ersten Position in die zweite Position bewegt wird, wenn sich das primäre Dosiseinstellelement (11) in einer Null-Einheiten-Dosisposition befindet, und derart, dass die Dosiswahlvorrichtung (12) in der zweiten Position drehfest am primären Dosiseinstellelement (11) gehalten wird und sich bezüglich des Gehäuses (4) frei drehen kann,
wobei eine Überbrückungskomponente (24) axial am sekundären Dosiseinstellelement (16) festgehalten wird und derart mit der Dosiswahlvorrichtung (12) verbunden ist, dass die Überbrückungskomponente (24) axial an der Dosiswahlvorrichtung (12) festgehalten wird, wenn die Dosiswahlvorrichtung (12) aus der ersten in die zweite Position bewegt wird, so dass eine Dosis des sekundären Medikaments eingestellt wird, und derart, dass eine Drehung der Dosiswahlvorrichtung (12) in die zweite Position zum Einstellen einer Dosis des primären Medikaments eine Bewegung der Dosiswahlvorrichtung (12) bezüglich der Überbrückungskomponente (24) in axialer Richtung verursacht.

2. Medikamenten-Verabreichungsvorrichtung nach Anspruch 1, wobei die Dosiswahlvorrichtung (12) als eine Hülse ausgelegt ist, die das primäre Dosiseinstellelement (11) zumindest teilweise umgibt.

3. Medikamenten-Verabreichungsvorrichtung nach Anspruch 1 oder 2, wobei die Dosiswahlvorrichtung (12) in der ersten Position axial am Gehäuse (4) verkeilt ist und in der zweiten Position am primären Dosiseinstellelement (11) verkeilt ist.

4. Medikamenten-Verabreichungsvorrichtung nach einem der vorhergehenden Ansprüche, wobei die Dosiswahlvorrichtung (12) ein Außengewinde (25) aufweist, das sich spiralförmig in axialer Richtung erstreckt und wobei die Überbrückungskomponente (24) mit dem Außengewinde (25) der Dosiswahlvorrichtung (12) in Gewindeeingriff (25a) steht.

5. Medikamenten-Verabreichungsvorrichtung nach einem der vorhergehenden Ansprüche,
wobei die sekundäre Medikamenten-Verabreichungsanordnung (3) eine Feder (27) umfasst, die dazu ausgelegt ist, das sekundäre Dosiseinstellelement (16) in proximaler Richtung vorzuspannen.

6. Medikamenten-Verabreichungsvorrichtung nach einem der vorhergehenden Ansprüche, wobei eine Sperre (26) zur Begrenzung der axialen Bewegung der Überbrückungskomponente (24) in proximaler Richtung bereitgestellt ist, wobei die Überbrückungskomponente (24) die Sperre (26) erreicht, wenn die Dosiswahlvorrichtung (12) die zweite Position erreicht.

7. Medikamenten-Verabreichungsvorrichtung nach einem der vorhergehenden Ansprüche, umfassend eine primäre Medikamentenkartusche (5) und eine sekundäre Medikamentenkartusche (6), wobei die primäre Medikamentenkartusche (5) mit der primären Medikamenten-Verabreichungsanordnung (2) verbunden ist und die sekundäre Medikamentenkartusche (6) mit der sekundären Medikamenten-Verabreichungsanordnung (3) verbunden ist, und wobei die primäre und die sekundäre Medikamentenkartusche (5, 6) jeweils ein Medikament enthalten.

8. Medikamenten-Verabreichungsvorrichtung nach einem der vorhergehenden Ansprüche, wobei die Medikamenten-Verabreichungsvorrichtung (1) als eine Einwegvorrichtung ausgelegt ist.

9. Medikamenten-Verabreichungsvorrichtung nach einem der vorhergehenden Ansprüche, wobei die sekundäre Medikamenten-Verabreichungsanordnung (3) eine Antriebshülse (19) umfasst, die lösbar mit dem sekundären Dosiseinstellelement (16) verbunden ist und mit einer Kolbenstange (20) verschraubt ist, so dass eine reine axiale Bewegung der Antriebshülse (19) eine Drehung der Kolbenstange (20) verursacht, wobei die Kolbenstange (20) mit dem Gehäuse oder einem Gehäuseeinsatz in Gewindeeingriff steht;
wobei eine Klickerfeder (28) vorgesehen ist, um die Antriebshülse (19) zur Abgabe einer Dosis drehfest festzuhalten, wobei die Klickerfeder (28) dazu ausgelegt ist, drehfest am Gehäuse festgehalten zu werden, wenn eine vorbestimmte Mindestdosis des sekundären Medikaments eingestellt wird.

10. Medikamenten-Verabreichungsvorrichtung nach einem der vorhergehenden Ansprüche, umfassend einen Dosisknopf (15), der in der Dosiswahlvorrichtung (12) derart angeordnet ist, dass eine Verschiebung des Dosisknopfes (15) in distaler Richtung eine Bewegung der Dosiswahlvorrichtung (12) in distaler Richtung aus der zweiten in die erste Position verursacht, und derart, dass, wenn sich die Dosiswahlvorrichtung (12) in der ersten Position befindet, der Dosisknopf (15) einen Abgabeknopf der primären Medikamenten-Verabreichungsanordnung (2) in Eingriff nimmt, so dass eine weitere Bewegung des Dosisknopfes (15) die Abgabe einer eingestellten Dosis des primären Medikaments verursacht.

## Revendications

1. Dispositif d'administration (1) de médicament comprenant :
un logement (4) retenant un ensemble d'administration (2) de médicament principal pour l'administration d'un médicament principal, l'ensemble d'administration (2) de médicament principal comprenant un élément de réglage (11) de dose principale conçu pour tourner dans un mouvement hélicoïdal dans une direction proximale (9) pour régler une dose du médicament principal ;
et un ensemble d'administration (3) de médicament secondaire pour l'administration d'un médicament secondaire, l'ensemble d'administration (3) de médicament secondaire comprenant un élément de réglage (16) de dose secondaire conçu pour se déplacer dans une direction proximale (9) pour régler une dose du médicament secondaire,
**caractérisé par** un cadran doseur (12) mobile entre une première position et une seconde position dans une direction axiale par rapport à l'élément de réglage (11) de dose principale, le cadran doseur (12) étant conçu de telle sorte que le cadran doseur (12) soit contraint en rotation par rapport au logement (4) lorsqu'il est déplacé depuis la première position vers la seconde position lorsque l'élément de réglage (11) de dose principale est dans une position de dose unitaire nulle et de telle sorte que le cadran doseur (12) soit contraint en rotation par rapport à l'élément de réglage (11) de dose principale dans la seconde position et soit libre de tourner par rapport au logement (4),
dans lequel un élément d'entretoise (24) est contraint axialement par rapport à l'élément de réglage (16) de dose secondaire et est accouplé au cadran doseur (12) de telle sorte que l'élément d'entretoise (24) soit contraint axialement par rapport au cadran doseur (12) lorsque le cadran doseur (12) est déplacé depuis la première vers la seconde position de sorte qu'une dose du médicament secondaire soit réglée, et de telle sorte que la rotation du cadran doseur (12) dans la seconde position pour régler une dose du médicament principal amène le cadran doseur (12) à se déplacer par rapport à l'élément d'entretoise (24) dans la direction axiale.

2. Dispositif d'administration de médicament selon la revendication 1, dans lequel le cadran doseur (12) est conçu sous la forme d'une gaine qui entoure au moins partiellement l'élément de réglage (11) de dose principale.

3. Dispositif d'administration de médicament selon la revendication 1 ou 2, dans lequel le cadran doseur (12) est claveté axialement sur le logement (4) dans la première position et est claveté sur l'élément de réglage (11) de dose principale dans la seconde position.

4. Dispositif d'administration de médicament selon l'une quelconque des revendications précédentes, dans lequel le cadran doseur (12) possède un filet externe (25) s'étendant hélicoïdalement dans une direction axiale et dans lequel l'élément d'entretoise (24) est en prise vissée (25a) avec le filet externe (25) du cadran doseur (12).

5. Dispositif d'administration de médicament selon l'une quelconque des revendications précédentes,
dans lequel l'ensemble d'administration (3) de médicament secondaire comprend un ressort (27) conçu pour solliciter l'élément de réglage (16) de dose secondaire dans une direction proximale.

6. Dispositif d'administration de médicament selon l'une quelconque des revendications précédentes, dans lequel une butée (26) est prévue pour limiter le déplacement axial de l'élément d'entretoise (24) dans une direction proximale, dans lequel l'élément d'entretoise (24) atteint la butée (26) lorsque le cadran doseur (12) atteint la seconde position.

7. Dispositif d'administration de médicament selon l'une quelconque des revendications précédentes comprenant une cartouche (5) de médicament principal et une cartouche (6) de médicament secondaire, dans lequel la cartouche (5) de médicament principal est accouplée à l'ensemble d'administration (2) de médicament principal et la cartouche (6) de médicament secondaire est accouplée à l'ensemble d'administration (3) de médicament secondaire, et dans lequel les cartouches (5, 6) de médicament principal et secondaire contiennent respectivement un médicament.

8. Dispositif d'administration de médicament selon l'une quelconque des revendications précédentes, dans lequel le dispositif d'administration (1) de médicament est conçu sous la forme d'un dispositif jetable.

9. Dispositif d'administration de médicament selon l'une quelconque des revendications précédentes, dans lequel l'ensemble d'administration (3) de médicament secondaire comprend une gaine d'entraînement (19) reliée de façon libérable à l'élément de réglage (16) de dose secondaire et vissée à une tige (20) de piston de sorte qu'un mouvement axial pur de la gaine d'entraînement (19) amène la tige (20) de piston à tourner, dans lequel la tige (20) de piston est en prise vissée avec le logement ou un insert du logement, dans lequel un ressort à cliquet (28) est prévu pour verrouiller en rotation la gaine d'entraînement (19) pour une distribution de dose, dans lequel le ressort à cliquet (28) est conçu pour être verrouillé en rotation sur le logement lorsqu'une dose minimum prédéfinie du médicament secondaire est réglée.

10. Dispositif d'administration de médicament selon l'une quelconque des revendications précédentes, comprenant un bouton doseur (15) agencé dans le cadran doseur (12) de sorte que le déplacement du bouton doseur (15) dans la direction distale amène le cadran doseur (12) à se déplacer dans la direction distale depuis la seconde position vers la première position et de sorte que lorsque le cadran doseur (12) est dans la première position, le bouton doseur (15) entre en prise avec un bouton distributeur de l'ensemble d'administration (2) de médicament principal de sorte qu'un mouvement supplémentaire du bouton doseur (15) amène une dose réglée du médicament principal à être distribuée.
